# EUROPEAN PATENT APPLICATION

(11) **EP 4 498 385 A1**
(43) Date of publication of application: **29.01.2025**
(21) Application number: 23188100.4
(22) Date of filing: 27.07.2023
(51) Int. Cl.: G16H 40/63, A61B 5/00, G06F 3/00, G06F 9/451, G16H 30/00, G09B 5/00

(54) **USER INTERFACE TRANSLATION APPARATUS AND METHOD**

(71) Applicant: Koninklijke Philips N.V., 5656 AG Eindhoven (NL)
(72) Inventor: WEISS, Steffen, Eindhoven (NL); VOGTMEIER, Gereon, Eindhoven (NL)
(74) Representative: Philips Intellectual Property & Standards

(57) **Abstract**

The invention relates to a user interface, UI, translation apparatus (2) for translating between a first UI of a medical imaging device (1) and a second UI, wherein the second UI is different from the first UI. The apparatus (2) comprises a first communication interface (6), a second communication interface (7) and a computing unit (8). The first communication interface (6) is adapted to receive first UI output (19) from the medical imaging device (1) and to send first UI input (22) to the medical imaging device (1), wherein the first UI output (19) and the first UI input (22) correspond to the first UI. The second communication interface (7) is adapted to send second UI output (21) to a UI arrangement (3) of the second UI and to receive second UI input (20) from the UI arrangement (3), wherein the second UI output (21) and the second UI input (20) correspond to the second UI. The computing unit (8) is configured to translate the first UI output (19) to the second UI output (21) and the second UI input (20) to the first UI input (22) such that the medical imaging device (1) is operatable using the second UI. The invention further relates to a related method (18) for translating between the first user UI and the second UI.

## Description

### FIELD OF THE INVENTION

The invention relates to medical imaging devices and, in particular, to a user interface (UI) translation apparatus for translating between a first UI of a medical imaging device and a second UI, wherein the second UI is different from the first UI. The invention further relates to a method for translating between a first UI of a medical imaging device and a second UI, wherein the second UI is different from the first UI.

### BACKGROUND OF THE INVENTION

User interfaces (UIs) in medical imaging are complex and differ from one another, in particular per vendor, since there exists no standardization of UIs. Even names and/or acronyms of imaging protocols, protocol parameters of sequences and technical subunits of the medical imaging devices differ from one another, even though the underlying medical imaging devices can be technically very similar to one another.

Staff using said UIs often have to switch between vendors and hence have little time to train and practice per UI type. Further, frequent updates of scan techniques via software releases, new hardware and related UI updates of all vendors add to a high learning effort.

It is essential for the health of patients that the staff operates the medical imaging devices correctly. Further, the staff must ensure that the imaging equipment is functioning properly, which can also be time-consuming and requires specialized knowledge.

Moreover, UIs of medical imaging devices are complex: planning and actual operation of medical imaging scans are performed via user input at said UIs with multiple hierarchical menus, buttons, controls, and settings. This can make it difficult to quickly and easily perform imaging procedures, leading to errors and lowering patient throughput especially for less experienced operators.

Further, a flexibility of switching from one medical imaging device to a different medical imaging device, e.g., when the staff has to temporarily work at a different hospital, is challenging due to the different UIs.

### SUMMARY OF THE INVENTION

It is therefore an object of the present invention to provide an apparatus and a corresponding method that overcome the above-mentioned problems. In particular, it is an object of the present invention to provide an apparatus and a corresponding method that translate between one user interface and a different user interface.

The object of the present invention is solved by the subject matter of the independent claims, wherein further embodiments are incorporated in the dependent claims.

In an aspect of the present invention, a user interface (UI) translation apparatus is provided. Said UI translation apparatus is adapted to translate between a first UI of a medical imaging device and a second UI, wherein the second UI is different from the first UI.

In this context "user interface" refers to both the UI hardware and the UI information. The UI hardware may be a UI arrangement which, for example, comprises a screen, a keyboard and a mouse. The UI information comprises the information exchanged between the medical imaging device and a user, in particular UI output, which is information exchanged from the medical imaging device to the user, and UI input, which is information that is input by the user and sent to the medical imaging device.

"Translating" between the first UI of the medical imaging device and the second UI means that the UI translation apparatus emulates the second UI for use on the medical imaging device.

While the second UI is different from the first UI, it is also equivalent to the first UI, i.e., it can provide at least some of, and preferably most of or all of, the functionality of the first UI.

The UI translation apparatus comprises a first communication interface, a second communication interface and a computing unit.

The first communication interface is adapted to receive first UI output from the medical imaging device and to send first UI input to the medical imaging device, wherein the first UI output and the first UI input correspond to the first UI. The first UI output and/or first UI input may be transferred using a wired and/or a wireless connection. For example, the first UI output may be transferred via an HDMI, DVI or VGA connection. As another example, the first UI input may be transferred via a USB connection.

The second communication interface is adapted to send second UI output to a UI arrangement of the second UI and to receive second UI input from the UI arrangement, wherein the second UI output and the second UI input correspond to the second UI. The UI arrangement is UI hardware corresponding to the second UI and comprises at least one output device, such as a screen and/or a speaker, and at least one input device, such as a mouse and/or a keyboard. The UI arrangement may be similar or identical to a UI arrangement corresponding to the first UI, in particular, when the UI arrangement comprises standard hardware such as a screen, a keyboard and a mouse, but may also be different from a UI arrangement corresponding to the first UI, in particular, if at least one of the UI arrangements comprises specialized hardware.

The computing unit is configured to translate the first UI output to the second UI output and the second UI input to the first UI input such that the medical imaging device is operatable using the second UI. In other words, the second UI output is UI output that is equivalent to the first UI output but mimics the second UI. Likewise, the first UI input is UI input that is equivalent to the second UI input but mimics the first UI. The first UI input is generated with respect to the first UI output, e.g., the mouse pointer location is relative to the screen output of the first UI. Effectively, the UI translation apparatus represents a layer between the UI arrangement operated by the user and a host computer of the medical imaging device. Hence, a user may only see and operate the second UI, while the medical imaging device works as if it was controlled using the first UI.

The UI translation apparatus therefore allows a user to operate the medical imaging device, that is usually operated via the first UI, via the second UI. Hence, the UI of the medical imaging device can be translated to a UI that the user is used to. In particular, UIs of different vendors can be translated into a single UI such that the user can operate many different medical imaging devices using a single UI. This reduces the learning effort of the user and improves both efficiency and reliability of the operation of the medical imaging device.

According to an embodiment, the medical imaging device is an ultrasound (US) imaging device, a digital X-ray (DXR) imaging device, a computed tomography (CT) imaging device and/or a magnetic resonance imaging (MRI) device. All of these medical imaging devices are operated using rather complex UIs, wherein usually the UIs are different for different vendors. Hence, the UI translation apparatus improves operation of said medical imaging devices for users who have to operate medical imaging devices from different vendors.

According to an embodiment, the second UI corresponds to a UI of another medical imaging device, wherein the other medical imaging device is of a different type than the medical imaging device. In particular, the other medical imaging device may be from a different vendor or from a different series of medical imaging devices than the medical imaging device. Hence, users who are used to the other medical imaging device may operate the medical imaging device using the UI of the other medical imaging device. Therefore, no extra training on the particulars of the medical imaging device is needed.

Alternatively, the second UI may be a UI that does not correspond to a specific medical imaging device. For example, the second UI may be optimized to ease translation between many different UIs and the second UI, e.g., by including the combined functionalities of many different UIs.

Yet alternatively, the second UI may correspond to the UI of another medical imaging device or even of the medical imaging device, but with limited functionalities, corresponding to a training level of a user. In particular, several different versions of the second UI may exist, each with a different set of functionalities.

According to an embodiment, the UI arrangement is a mobile UI arrangement. For example, the mobile UI arrangement may comprise a tablet computer or a smartphone.

According to an embodiment, the UI translation apparatus further comprises a third communication interface that is adapted to output the first UI output to another UI arrangement. As an example, a screen output of the first UI output, i.e., the original screen output from the medical imaging device, may be displayed on a second screen. The user can use this to confirm that any input that was done using the second UI is correctly transferred into the first UI. Further, e.g., for training purposes, the user can see how his or her actions would look on the first UI. Moreover, the second screen can be used as viewing station for the diagnostic image to have a high quality display.

Alternatively, the third communication interface may be adapted to provide another instance of the second UI. This can be used, e.g., for training purposes or for remote support. For example, the second communication interface may be connected to a UI arrangement in the vicinity of the medical imaging device whereas the third communication interface may be connected to a remote UI arrangement.

Yet alternatively, the third communication interface may be connected to a mobile UI arrangement whereas the second communication interface may be connected to a stationary UI arrangement. Hence, a user may operate the medical imaging device using both, the mobile UI arrangement and the stationary UI arrangement.

According to an embodiment, the first UI output and/or the second UI output comprise video output and/or audio output. In particular, the video output may include output of images obtained by the medical imaging device as well as menus, buttons, entry forms, etc., used to operate the medical imaging device. The video output may be provided, e.g., via an HDMI connection, a DVI connection or a VGA connection, but also via a machine interface. The audio output may, e.g., include alert sounds and may be provided via an electrical or optical audio connection.

According to an embodiment, the first UI input and/or the second UI input comprise mouse input and/or keyboard input. These UI inputs may be provided, e.g., via a USB connection or other accessible interfaces.

According to an embodiment, the step of translating the first UI output to the second UI output and the second UI input to the first UI input comprises analyzing the first UI output and analyzing the second UI input.

Analyzing the first UI output means, in particular, analyzing the structure and the content of the first UI output. As an example, a text recognition system and/or an optical character recognition system may be used to detect text and coordinates of the text in a video output of the first UI output. Further, an image processor may be used to grab images, in particular results of the medical imaging procedure and/or graphics relating to the medical imaging device, in the video output of the first UI output. Said image processor may be a conventional image processor, using, e.g., edge detection to find the corners of the images in the video output. When grabbing the images, special care may have to be taken for grayscales and dynamic range having a good image quality. This may be achieved, e.g., by calibrating the image grabber with test images. Optionally, the images that have been grabbed from the video output may be further analyzed to extract additional elements or information. As an example, graphical elements overlayed on a medical image shown in the video output of the first UI output as lines or rectangles as commonly used to define the field of view of a diagnostic scan based on the content of a survey or localizer scan may be analyzed. As a second example, symbols as arrows or dedicated icons that support image manipulation as zooming or level/window may be analyzed. Also, the audio output of the first UI output may be analyzed, e.g., by comparing the received sounds, which often are alert sounds, to a known set of sounds.

The second UI input may be analyzed in relation to the second UI output. In particular, a position of a mouse cursor on the screen has to be known in relation to currently displayed widgets such as menus, buttons, and text input fields in order to determine the action of a click with the mouse. Said analysis may be performed using parts of the original software of the other medical imaging device, if the second UI corresponds to a UI of the other medical imaging device.

According to an embodiment, the step of translating the first UI output to the second UI output and the second UI input to the first UI input is performed using two UI state machines, one for the first UI and one for the second UI. In detail, a first UI state machine is set up, modelling states of the first UI, and a second UI state machine is set up, modelling states of the second UI. Said state machines may comprise a complete state of the UI, including currently displayed menus, submenus, buttons, checkmarks, text input fields including their states as whether a checkmark is set or not, and the text of text input fields. The state of the UI may further comprise numbers, values, flags, images, error messages, system warnings, maintenance routines and/or calibration routines and may include information that is not currently visible in the UI video but hidden in other menus.

The state of the first UI state machine is changed based on the analyzed first UI output. In particular, when changes in the first UI output are detected, those changes are converted into changes of the first UI state machine. The current state of the first UI state machine may also be used as input for the analysis of the video output of the first UI output: usually, in a given state, only a finite number of state changes may occur, e.g., by text appearing on the screen, a menu popping up, or a check mark being checked or unchecked. Hence, in the analysis, only a low-dimensional and finite space has to be searched, which simplifies the classification.

Further, the state of the second UI state machine is changed, based on the analyzed second UI input.

The first UI state machine and the second UI state machine are then kept in equivalent and/or associated states by transferring any change occurring in one of the state machines into the other one of the state machines. More particularly, a change in the first UI state machine is transferred to the second UI state machine and a change in the second UI state machine is transferred to the first UI state machine. In this context, equivalent and/or associated states are states that most closely resemble the state of the other UI state machine. This includes, for example, using the equivalent names for, e.g., MR sequences or for MR scan protocol parameters. Further, since medical imaging devices of a different type may have different hardware specifications, it may be necessary to define modified scans to compensate this difference. The respective logic and/or association of said UI states is part of the step of translating the first UI output to the second UI output and the second UI input to the first UI input. As an example, in CT, different widths of CT detectors or a different table pitch have to be compensated for. As another example, in MRI, a different radio frequency peak power, and/or different gradient strength and slew rate have to be compensated for, e.g., by choosing correct timing of the MR sequences. If it is not possible to find equivalent and/or associated first UI states for a second UI state, said second UI state may be disabled such that it cannot be chosen by the user. On the other hand, if it is not possible to find equivalent and/or associated second UI states for a first UI state, an alert or an error message may be issued.

Then, the second UI output is generated based on the changes made to the second UI state machine. This generation may include encoding of the layout and widgets of the second UI. The first UI input is generated based on the changes made to the first UI state machine. Hence, translating between the first UI and the second UI is enabled in a powerful way by using the state machines.

If the second UI corresponds to the UI of the other medical imaging device, generating the second UI output given the second UI state machine may be performed using genuine software or parts of the genuine software of the other medical imaging device.

According to an embodiment, changing the state of the first UI state machine based on the analyzed first UI output comprises using conventional classifiers to map text and/or images to changes of the first UI state machine. In particular, said conventional classifiers may be provided by a human, in particular by a medical imaging specialist who is trained on the medical imaging device and experienced with the second UI.

Alternatively, or additionally, changing the state of the first UI state machine based on the analyzed first UI output comprises using neural networks to map the video output of the first UI or text and/or images thereof to changes of the first UI state machine. Such a neural network may be trained, e.g., by operating the medical imaging device with the first UI and a second UI in parallel, performing the actions on the second UI that correspond to the actions on the first UI. In this way, training data is generated, giving the equivalent state changes for the first UI and the second UI. With this training data, the neural networks are trained.

Optionally, both the conventional classifiers and/or the neural networks may use, in addition to the first UI input, first UI output, second UI input and second UI output, the current state of the first UI state machine as input parameter, to improve the learning and the accuracy of the conventional classifiers and/or the neural networks.

According to another aspect of the invention, a method for translating between a first user interface (UI) of a medical imaging device and a second UI, wherein the second UI is different from the first UI, is provided.steffen.weisshilips.com

In this context "user interface" refers to both the UI hardware and the UI information. The UI hardware may be a UI arrangement which, for example, comprises a screen, a keyboard and a mouse. The UI information comprises the information exchanged between the medical imaging device and a user, in particular UI output, which is information exchanged from the medical imaging device to the user, and UI input, which is information that is input by the user and sent to the medical imaging device.

"Translating" between the first UI of the medical imaging device and the second UI means that the UI translation apparatus emulates the second UI for use on the medical imaging device.

While the second UI is different from the first UI, it is also equivalent to the first UI, i.e., it can provide at least some of, and preferably most of or all of, the functionality of the first UI.

According to the method, first UI output is received from the medical imaging device, wherein the first UI output corresponds to the first UI. The first UI output may be received using a wired and/or a wireless connection. For example, the first UI output may be received via an HDMI, DVI or VGA connection.

Further, second UI input is received from a UI arrangement of the second UI, wherein the second UI input corresponds to the second UI and the UI arrangement comprises at least one output device, such as a screen and/or a speaker, and at least one input device, such as a mouse and/or a keyboard. The UI arrangement may be similar or identical to a UI arrangement corresponding to the first UI, in particular, when the UI arrangement comprises standard hardware such as a screen, a keyboard and a mouse, but may also be different from a UI arrangement corresponding to the first UI, in particular, if at least one of the UI arrangements comprises specialized hardware.

The first UI output is translated to the second UI output and the second UI input is translated to the first UI input such that the medical imaging device is operatable using the second UI. Here, the second UI output corresponds to the second UI and the first UI input corresponds to the first UI. Specifically, the second UI output is UI output that is equivalent to the first UI output but mimics the second UI. Likewise, the first UI input is UI input that is equivalent to the second UI input but mimics the first UI. The first UI input is generated with respect to the first UI output, e.g., the mouse pointer location is relative to the screen output of the first UI. Hence, a user may only see the second UI, while the medical imaging device works as if it was controlled using the first UI.

Then, the first UI input is sent to the medical imaging device and the second UI output is sent to the UI arrangement. Here, the first UI input may be transferred using a wired and/or a wireless connection. For example, the first UI input may be transferred via a USB connection.

By translating between the first UI and the second UI, a user is allowed to operate the medical imaging device, that is usually operated via the first UI, via the second UI. Hence, the UI of the medical imaging device can be translated to a UI that the user is used to. In particular, UIs of different vendors can be translated into a single UI such that the user can operate many different medical imaging devices using a single UI. This reduces the learning effort of the user and improves both efficiency and reliability of the operation of the medical imaging device.

According to an embodiment, a setup check is performed. Said setup check may be performed at the beginning of a medical imaging routine and/or periodically. The setup check is performed by sending first UI input corresponding to accessing a configuration and/or version page to the medical imaging device and analyzing the resulting first UI output to obtain the provided setup information.

According to an embodiment, first UI input that initiates a calibration run of the medical imaging device is generated and sent to the medical imaging device. Hence, with this method step, a calibration run of the medical imaging device is initiated. This calibration run may be performed, e.g., at the beginning of each medical imaging session, or before and/or after each medical imaging run. Similarly, a calibration of the UI arrangement, e.g., of a color space of a screen and/or a movement range of a mouse, may be performed.

According to an embodiment, a training function is provided to users of the medical imaging device. Said training function may, depending on the user's experience, provide only a set of available functions. In particular, for a beginner, the training function may provide only the most basic functions to the user, whereas for an experienced user, the training function may provide all of the available functions. In other words, the training function may provide a curriculum that introduces the user first to basic and then to more complex functions of the UI. This may be supported by displaying only core functions in the beginning and hinting to more complex functions for advanced learners. Further, the training function may keep book of how often certain functions have been used and trigger training of functions that have rarely been used.

According to an embodiment, efficiency and/or ergonomics of the first UI and of the second UI are measured and a comparison of the efficiency and/or ergonomics of the first UI and the second UI is provided. Said measurement may be performed, in particular, based on the translation between the first UI and the second UI, since equivalent actions are performed by the first UI and the second UI. Measures for efficiency and/or ergonomics may be the number of mouse clicks and/or drags required to define and/or plan a certain scan, and/or the number of menus and/or tabs that have to be gone through.

It shall be understood that a preferred embodiment of the invention can also be any combination of the dependent claims with the respective independent claim. Further, features that have only been described for the apparatus may also apply to the method and vice versa.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematic view of an embodiment of a medical imaging device with a user interface (UI) translation apparatus;
Fig. 2a shows a schematic screen output of an example of a UI;
Fig. 2b shows a schematic screen output of another example of a UI;
Fig. 3 shows a flowchart of an embodiment of a method for UI translation; and
Fig. 4 shows a flowchart of another embodiment of a method for UI translation.

In the Figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

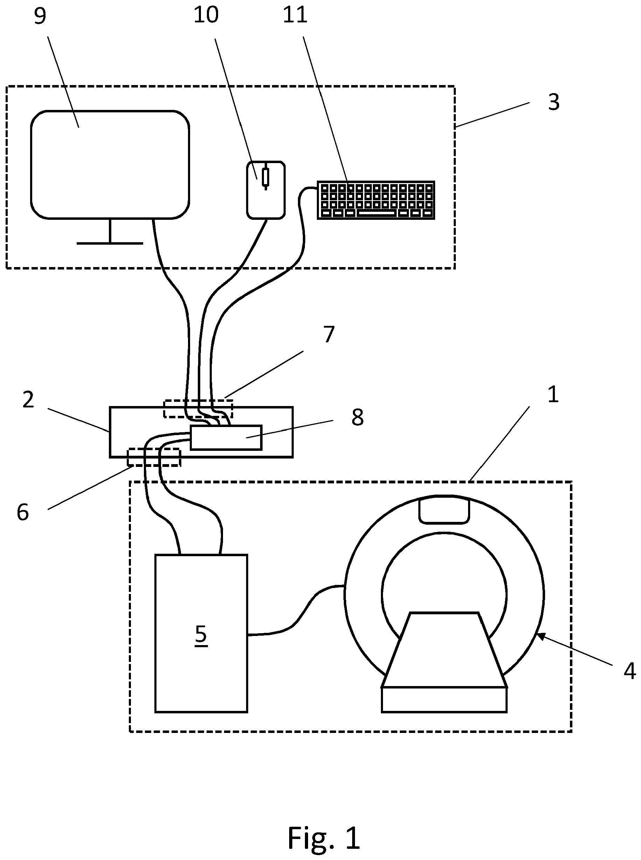

Fig. 1 shows a schematic view of an embodiment of a medical imaging device 1 with a user interface (UI) translation apparatus 2 and a UI arrangement 3.

The medical imaging device 1 may be an ultrasound imaging device, a digital X-ray imaging device, a computed tomography imaging device and/or a magnetic resonance imaging device and comprises a medical imaging apparatus 4 and a host computer 5. The medical imaging apparatus 4 and the host computer 5 may be separate entities, as shown in Fig. 1, connected by a wired or wireless connection. Alternatively, the host computer 5 may be integrated in the medical imaging apparatus 4 to form the medical imaging device 1.

The UI translation apparatus 2 comprises a first communication interface 6, a second communication interface 7 and a computing unit 8.

The first communication interface 6 is adapted to receive first UI output from the medical imaging device 1, in particular from the host computer 5 of the medical imaging device 1, and to send first UI input to the medical imaging device 1. Here, the first UI output and the first UI input correspond to a first UI, wherein the first UI is the UI of the medical imaging device 1.

The second communication interface 7 is adapted to send second UI output to the UI arrangement 3 and receive second UI input from the UI arrangement 3. Here, the second UI output and the second UI input correspond to a second UI, wherein the second UI is different from the first UI. While the second UI is different from the first UI, it is also equivalent to the first UI, i.e., it can provide at least some of, and preferably most of or all of, the functionality of the first UI.

The UI arrangement 3 is shown as comprising a screen 9 as an output device and a mouse 10 and a keyboard 11 as input devices. However, also other output devices such as speakers or other input devices may be part of the UI arrangement 3. Further, the UI arrangement 3 may comprise separate output and input devices and/or may comprise an integrated output/input device.

The computing unit 8 is configured to translate between the first UI and the second UI. In particular, the computing unit 8 is configured to translate the first UI output to the second UI output and the second UI input to the first UI input such that the medical imaging device 1 is operatable using the second UI. In other words, the second UI output is UI output that is equivalent to the first UI output but mimics the second UI. Likewise, the first UI input is UI input that is equivalent to the second UI input but mimics the first UI. The first UI input is generated with respect to the first UI output, e.g., the mouse pointer location is relative to the screen output of the first UI. Effectively, the UI translation apparatus 2 represents a layer between the UI arrangement 3 operated by the user and the host computer 5 of the medical imaging device 1. Hence, a user may only see and operate the second UI, while the medical imaging device 1 works as if it was controlled using the first UI.

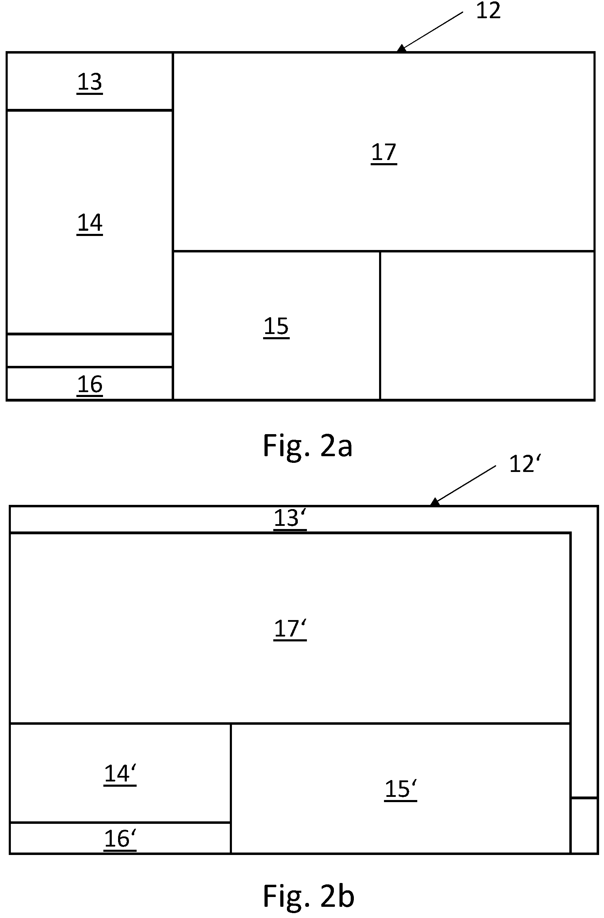

Fig. 2a shows a schematic screen output 12 of an example of a UI, e.g., the screen output corresponding to the first UI. Said screen output 12 comprises a menu 13, a window 14 containing information on a scan sequence, a window 15 containing information on scan parameters, an area 16 comprising widgets to control a scan as well as a window 17 showing the images obtained by the medical imaging device 1.

Fig. 2b shows a schematic screen output 12' of another example of a UI, e.g., the screen output corresponding to the second UI. Similar to the screen output 12, the screen output 12' comprises a menu 13', a scan sequence window 14', a scan parameter window 15', a control area 16' and an image window 17'.

As can be easily seen, the screen output 12 of the example UI and the screen output 12' of the other example UI are similar to one another. However, it has to be noted that the content comprised in the separate windows may also be similar, but not identical. Hence, by translating between the first UI and the second UI, a user may operate the medical imaging device 1, that is usually operated via the first UI, via the second UI. That is, the UI of the medical imaging device 1 can be translated to a UI that the user is used to. In particular, UIs of different vendors can be translated into a single UI such that the user can operate many different medical imaging devices 1 using a single UI. This reduces the learning effort of the user and improves both efficiency and reliability of the operation of the medical imaging device 1.

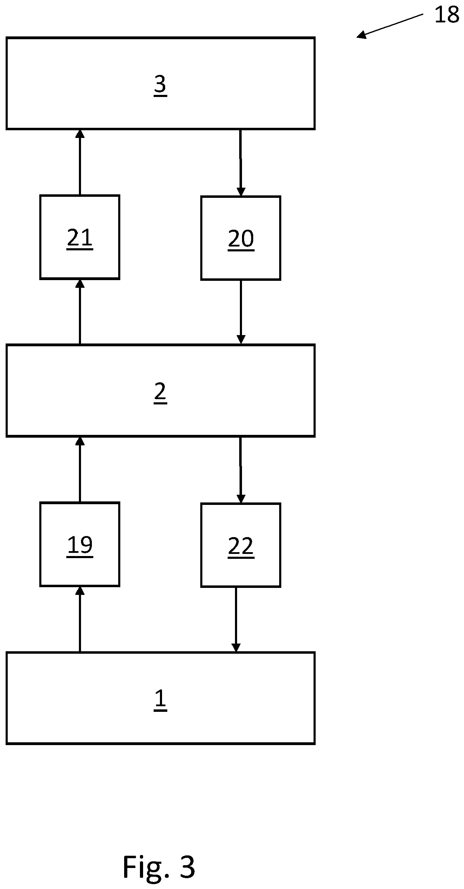

Fig. 3 shows a flowchart of an embodiment of a method 18 for UI translation. According to said method, first UI output 19 is received by the UI translation apparatus 2 from the medical imaging device 1. Further, second UI input 20 is received from the UI arrangement 3. The UI translation apparatus 2 then translates the first UI output 19 to second UI output 21 and the second UI input 20 to first UI input 22, sends the first UI input 22 to the medical imaging device 1 and the sends the second UI output 21 to the UI arrangement 3. The translation of the first UI output 19 to the second UI output 21 and the second UI input 20 to the first UI input 22 is performed such that the medical imaging device 1 is operatable using the second UI.

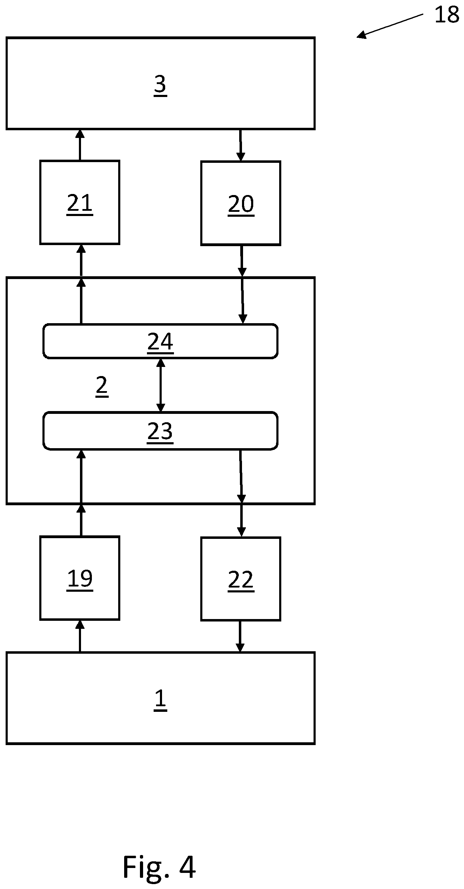

Fig. 4 shows a flowchart of another embodiment of a method 18 for UI translation. In particular, compared to the method 18 shown in Fig. 3, more details of the translation are provided. More particularly, the UI translation apparatus 2 sets up a first UI state machine 23 that models states of the first UI and a second UI state machine 24 that models states of the second UI. Said state machines may comprise a complete state of the UI, including currently displayed menus, submenus, buttons, checkmarks, text input fields including their states as whether a checkmark is set or not, and the text of text input fields. The state of the UI may further comprise numbers, values, flags, images, error messages, system warnings, maintenance routines and/or calibration routines and may include information that is not currently visible in the UI video but hidden in other menus.

The state of the first UI state machine 23 is then changed based on analyzed first UI output 19 and the state of the second UI state machine 24 is changed based on analyzed second UI input 20. The first UI state machine 23 and the second UI state machine 24 are kept in equivalent and/or associated states by transferring any change occurring in one of the state machines into the other one of the state machines, as indicated by the double arrow between the state machines. More particularly, a change in the first UI state machine is transferred to the second UI state machine and a change in the second UI state machine is transferred to the first UI state machine. Here, equivalent and/or associated states are states that most closely resemble the state of the other UI state machine. This includes, for example, using the equivalent names for, e.g., MR sequences or for MR scan protocol parameters. Further, since medical imaging devices of a different type may have different hardware specifications, it may be necessary to define modified scans to compensate this difference. The respective logic and/or association of said UI states is part of the step of translating the first UI output 19 to the second UI output 21 and the second UI input 20 to the first UI input 22. As an example, in CT, different widths of CT detectors or a different table pitch have to be compensated for. As another example, in MRI, a different radio frequency peak power, and/or different gradient strength and slew rate have to be compensated for, e.g., by choosing correct timing of the MR sequences. If it is not possible to find equivalent and/or associated first UI states for a second UI state, said second UI state may be disabled such that it cannot be chosen by the user. On the other hand, if it is not possible to find equivalent and/or associated second UI states for a first UI state, an alert or an error message may be issued.

Further, the second UI output 21 is generated based on the changes made to the second UI state machine 24 and the first UI input 22 is generated based on the changes made to the first UI state machine 23.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: medical imaging device
- 2: UI translation apparatus
- 3: UI arrangement
- 4: medical imaging apparatus
- 5: host computer
- 6: first communication interface
- 7: second communication interface
- 8: computing unit
- 9: screen
- 10: mouse
- 11: keyboard
- 12: screen output
- 13: menu
- 14: scan sequence window
- 15: scan parameter window
- 16: control area
- 17: image window
- 18: UI translation method
- 19: first UI output
- 20: second UI input
- 21: second UI output
- 22: first UI input

## Claims

1. User interface, UI, translation apparatus (2) for translating between a first UI of a medical imaging device (1) and a second UI, wherein the second UI is different from the first UI, the apparatus (2) comprising:
a first communication interface (6) adapted to receive first UI output (19) from the medical imaging device (1) and to send first UI input (22) to the medical imaging device (1), wherein the first UI output (19) and the first UI input (22) correspond to the first UI;
a second communication interface (7) adapted to send second UI output (21) to a UI arrangement (3) of the second UI and to receive second UI input (20) from the UI arrangement (3), wherein the second UI output (21) and the second UI input (20) correspond to the second UI and the UI arrangement (3) comprises at least one output device (9) and at least one input device (10; 11); and
a computing unit (8) configured to translate the first UI output (19) to the second UI output (21) and the second UI input (20) to the first UI input (22) such that the medical imaging device (1) is operatable using the second UI.

2. UI translation apparatus (2) according to claim 1, wherein the medical imaging device (1) is an ultrasound imaging device, a digital X-ray imaging device, a computed tomography imaging device and/or a magnetic resonance imaging device.

3. UI translation apparatus (2) according to claim 1 or 2, wherein the second UI corresponds to a UI of another medical imaging device, wherein the other medical imaging device is of a different type than the medical imaging device (1).

4. UI translation apparatus (2) according to any one of claims 1 to 3, wherein the UI arrangement (3) is a mobile UI arrangement.

5. UI translation apparatus (2) according to any one of claims 1 to 4, further comprising a third communication interface, adapted to output the first UI output (19) to another UI arrangement.

6. UI translation apparatus (2) according to any one of claims 1 to 5, wherein the first UI output (19) and/or the second UI output (21) comprise video output and/or audio output.

7. UI translation apparatus (2) according to any one of claims 1 to 6, wherein the first UI input (22) and/or the second UI input (20) comprise mouse input and/or keyboard input.

8. UI translation apparatus (2) according to any one of claims 1 to 7, wherein translating the first UI output (19) to the second UI output (21) and the second UI input (20) to the first UI input (22) comprises:
analyzing the first UI output (19), for example,
using a text recognition system to detect text and coordinates of the text in a video output; and/or
using an image processor to grab and/or analyze images in the video output; and
analyzing the second UI input (20).

9. UI translation apparatus (2) according to claim 8, wherein translating the first UI output (19) to the second UI output (21) and the second UI input (20) to the first UI input (22) comprises:
setting up a first UI state machine modelling states of the first UI;
setting up a second UI state machine modelling states of the second UI;
changing the state of the first UI state machine based on the analyzed first UI output (19);
changing the state of the second UI state machine based on the analyzed second UI input (20);
keeping the first UI state machine and the second UI state machine in equivalent and/or associated states by transferring any change occurring in one of the state machines into the other one of the state machines;
generating the second UI output (21) based on the changes made to the second UI state machine; and
generating the first UI input (22) based on the changes made to the first UI state machine.

10. UI translation apparatus (2) according to claim 9, wherein changing the state of the first UI state machine based on the analyzed first UI output (19) comprises using conventional classifiers and/or neural networks to map text and/or images to changes of the first UI state machine, wherein, more particularly, the conventional classifiers and/or neural networks use the current state of the first UI state machine as input parameter.

11. Method (18) for translating between a first user interface, UI, of a medical imaging device (1) and a second UI, wherein the second UI is different from the first UI, the method (18) comprising:
receiving first UI output (19) from the medical imaging device (1), wherein the first UI output (19) corresponds to the first UI;
receiving second UI input (20) from a UI arrangement (3) of the second UI, wherein the second UI input (20) corresponds to the second UI and the UI arrangement (3) comprises at least one output device (9) and at least one input device (10; 11);
translating the first UI output (19) to the second UI output (21) and the second UI input (20) to the first UI input (22), wherein the second UI output (21) corresponds to the second UI and the first UI input (22) corresponds to the first UI, such that the medical imaging device (1) is operatable using the second UI;
sending the first UI input (22) to the medical imaging device (1); and
sending the second UI output (21) to the UI arrangement (3).

12. The method (18) according to claim 11, further comprising:
performing a setup check by sending first UI input (22) corresponding to accessing a configuration and/or version page to the medical imaging device (1) and analyzing the resulting first UI output (19).

13. The method (18) according to claim 11 or 12, further comprising:
generating first UI input (22) that initiates a calibration run of the medical imaging device (1); and
sending the generated first UI input (22) to the medical imaging device (1).

14. The method (18) according to any one of claims 11 to 13, further comprising:
providing a training function to users of the medical imaging device (1).

15. The method (18) according to any one of claims 11 to 14, further comprising:
measuring efficiency and/or ergonomics of the first UI and of the second UI; and
providing a comparison of the efficiency and/or ergonomics of the first UI and the second UI.
